# EUROPEAN PATENT SPECIFICATION

(11) **EP 3 137 563 B1**
(45) Date of publication and mention of the grant of the patent: **31.08.2022**
(21) Application number: 15718501.8
(22) Date of filing: 28.04.2015
(51) Int. Cl.: C09D 175/06, C09D 175/16, C08G 18/75, C08G 18/42, C08G 18/44, C08G 18/67, C08G 18/08, C08G 18/12, A61Q 3/02

(54) **NAIL POLISH COMPOSITION BASED ON SOLVENT-FREE AQUEOUS POLYURETHANE DISPERSIONS**
NAGELLACKZUSAMMENSETZUNG AUF BASIS VON LÖSUNGSMITTELFREIEN WÄSSRIGEN POLYURETHANDISPERSIONEN
COMPOSITION DE VERNIS À ONGLES À BASE DE DISPERSIONS AQUEUSES DE POLYURÉTHANE EXEMPTES DE SOLVANT

(30) Priority: 30.04.2014 US 201461986165 P
(43) Date of publication of application: 08.03.2017
(73) Proprietor: Arkema France, 92700 Colombes (FR)
(72) Inventor: KLANG, Jeffrey, West Chester, Pennsylvania 19382 (US); LU, Jin, West Chester, Pennsylvania 19382 (US); VAPPALA, Indu, Exton, Pennsylvania 19341 (US)
(74) Representative: Arkema Patent
(86) International application number: PCT/EP2015/059196
(87) International publication number: WO 2015/165897

(56) References cited:
- WO-A1-2015/075193
- WO-A2-01/66659
- WO-A2-2006/138557
- US-A- 5 716 603
- US-A1- 2004 254 293
- US-A1- 2005 192 400
- US-A1- 2006 251 896
- US-A1- 2010 119 735
- US-A1- 2010 210 757
- US-A1- 2012 041 145
- US-A1- 2012 259 065
- US-A1- 2013 041 072

## Description

The present invention relates to the use in nail polish compositions of specific aqueous polyurethane polymer dispersions. More specifically, the present invention relates to the use nail polish compositions of solvent-free aqueous polyurethane dispersions that are curable by actinic radiation.

Polyurethane dispersions find many uses in industry. For example, polyurethane dispersions may be used to coat wood, plastic, metal, glass, fibers, textiles, leather, stone, concrete and other substrates to provide protection against mechanical, chemical and/or environmental effects. Polyurethane dispersions may also be used for adhesives, sealants, inks and other applications, including cosmetic applications, such as nail polishes and the like.

Polyurethane coatings formed from polyurethane dispersions may be used to provide scratch, abrasion and wear resistance ; UV protection ; corrosion resistance ; glossy or flat appearance ; chemical and stain resistance ; hydrolytic resistance ; flame retardancy ; anti-microbial activity ; electrical conduction or insulation ; barrier or permeability to gasses ; adhesion ; haptic effects such as soft touch ; easy cleaning and anti-fingerprint.

Polyurethane dispersions are typically produced by first forming a polyurethane pre-polymer, which comprises terminal groups, such as isocyanate (NCO) groups, which can undergo subsequent chain extension reactions. To facilitate the formation of the dispersion, the polyurethane pre-polymer is usually diluted with an organic solvent before dispersion in water to lower the viscosity of the pre-polymer. The pre-polymer can undergo a chain-extension reaction prior to or after dispersion in water to increase the length of the polymer chain and/or add additional functionality to the polyurethane.

The use of organic solvents may be undesirable due to volatility, flammability and the difficulty in removing the organic solvents from the polyurethane dispersions. There is also increasing pressure for industrial processes to minimize the use of volatile organic compounds (VOCs). Many VOCs have been linked to environmental issues and may be hazardous to workers exposed to them.

The organic solvent is usually removed after dispersion in water. Distillation is a typical method for removing the organic solvent, but other methods may also be used. Acetone is a common solvent used in the preparation of polyurethane dispersions. Due to cost, acetone is often recycled for repeated use. However, wet acetone, i.e., acetone containing water, cannot be reused in polyurethane production processes. The separation of acetone in the polyurethane production adds cost, complexity and time. Due to the difficulty of removal, at least some acetone is expected to remain in the polyurethane dispersion.

U.S. Patent Application Publication No. 2002/0259065 discloses a process for preparing isocyanate-terminated pre-polymers with low viscosity. The pre-polymers are formed using a specific reaction sequence in which a reaction of NCO-functionalized compounds with OH-functionalized compounds initially takes place in the absence of acid-carrying compounds. The isocyanate-terminated pre-polymer is formed in acetone solvent, which is later removed by distillation.

WO 2012/126911 discloses a process for forming polyurethane dispersions which contain polyurethane pre-polymers having a low enough viscosity such that they can be dispersed without the use of solvents or other diluents. However, the process uses hydroxyl terminal groups rather than isocyanate terminal groups, which limit the functionality of the polyurethanes. The pre-polymers are also not acrylated and cannot be UV curable.

U.S. Patent No. 6,372,201 discloses a nail varnish containing an aqueous dispersion of particles of acrylic polymer, a first organic solvent having a boiling point greater than or equal to 225°C and a second organic solvent having a boiling point ranging from 70°C to 180°C.

U.S. Patent No. 6,267,950 discloses a nail varnish comprising an aqueous dispersion of a film-forming polymer and an associative polyurethane. The associative polyurethane is a nonionic block copolymer comprising hydrophilic sequences and hydrophobic sequences. The film-forming polymer may be chosen from free radical polymers, polycondensates and polymers of natural origin.

U.S. Patent No. 5,716,603 discloses an aqueous acrylic resin crosslinked with difunctional acrylated urethane oligomers for use in nail polish compositions. The acrylic resin crosslinked with difunctional acrylated urethane oligomers is formed by the polymerization of : (1) a difunctional acrylated urethane oligomer; (2) an α,β-ethylenically unsaturated carboxylic acid monomer containing 3 to 10 carbon atoms ; (3) an acrylate ester of a specified formula and (4) a methacrylate ester of a specified formula.

U.S. Patent Application No. 2007/0243149 discloses an aqueous binder system for nail varnishes based on nitrocellulose-containing polyurethane polyurea dispersions.

Further information is available in U.S. Patent No. 5,596,065 and publication WO 2012/089538 A1.

Therefore, it is desirable to provide a process for producing polyurethane dispersions in a solvent-free process. It is also desirable to provide isocyanate terminated polyurethane pre-polymers to provide functional flexibility to the formed polyurethane dispersions.

The present invention is as defined in the claims.

The present invention relates to the use in nail polish compositions of specific aqueous polyurethane dispersions and methods of producing aqueous polyurethane dispersions.

One aspect of the present invention relates to the use in a nail polish composition, in particular a nail polish formulation, of at least one aqueous, actinic radiation curable polyurethane dispersion, which dispersion is free of non-reactive solvent and comprises a polyurethane polymer in at least one reactive diluent (F) and the said polyurethane polymer, in particular bearing ethylenic unsaturation end-groups and optionally side groups, is formed by chain extending at least one isocyanate-terminated ethylenically unsaturated polyurethane pre-polymer (P), in particular with at least one chain extender (E) bearing at least 2 isocyanate-reactive groups and wherein said pre-polymer is formed by reacting :
(A) one or more isocyanate-reactive components containing at least one ethylenic unsaturation chosen from active hydrogen-containing (meth)acrylates
(B) one or more polyisocyanates, preferably diisocyanates ;
(C) one or more isocyanate reactive components containing ionic groups, potentially ionic groups or hydrophilic ether groups, preferably ionic groups derived from acidic groups, in particular with said component (C) bearing two isocyanate-reactive groups, more particularly bearing two OH groups ; and
(D) optionally, one or more isocyanate-reactive components other than component (A) or component (C), preferably bearing two isocyanate-reactive groups, in particular OH groups,
such that the mole ratios of components (A), (B), (C) and (D) result in a polyurethane pre-polymer comprising terminal isocyanate group.

Said composition may be a nail polish formulation and in addition to said aqueous polyurethane polymer dispersion said formulation further comprises :
b) a photoinitiator
c) optionally, a leveling agent and
d) optionally, a thickener,
and wherein said formulation is free of non-reactive solvents.

In addition, the present invention relates to the use of said nail polish composition or nail polish formulation or of said aqueous polyurethane polymer dispersion for coating nails.

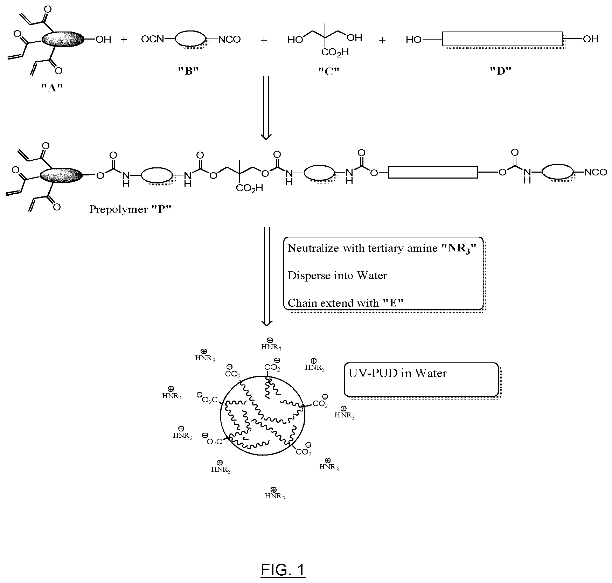

Figure 1 is a schematic of a synthesis process for forming a UV-curable polyurethane dispersion using a polyol (D), which polyurethane dispersion is suitable for the said nail polish composition as defined according to the present invention.

One aspect of the present disclosure relates to a composition free of non-reactive solvents comprising a polyurethane polymer, derived from the said polyurethane pre-polymer (P) and said reactive diluent (F).

As used herein, the term "pre-polymer" refers to an ethylenically unsaturated compound that comprises one or more isocyanate terminal groups. The pre-polymer is reacted with a chain extender (E), containing at least two isocyanate-reactive groups, in particular OH groups reacting with said pre-polymer in a chain extension reaction.

As used herein, the term "reactive diluent" refers to a compound (F) having two or more ethylenically unsaturated groups that may be used as a diluent in the preparation of the polyurethane pre-polymer and to dilute the polyurethane pre-polymer P during the formation of the polyurethane dispersion. It may react by free radical reaction with the (meth)acrylate groups on dispersed polyurethane during the actinic radiation curing step. The reactive diluent (F), when added to the polyurethane pre-polymer (P), can be used to control the viscosity of the polyurethane pre-polymer (P).

As used herein, the phrase "free of non-reactive solvent" and variations thereof means that a non-reactive solvent is not present (a non-reactive solvent is absent) in any amount. The term "non-reactive solvent" refers to solvents or diluents, other than water, which do not form part of the cured polyurethane. Compositions free of non-reactive solvent according to the present disclosure do not include trace amounts of non-reactive solvents which remain in processes which use non-reactive solvents that are subsequently removed by distillation or other processes for removing the non-reactive solvents. The phrase "substantially free of non-reactive solvent" means that trace amounts of non-reactive solvent are present in compositions of the invention, e.g., less than 1%, preferably less than 0.5%, more preferably less than 0.2%, most preferably less than 0.1%, based on the total weight of the composition. More particularly, there is 0% content of non-reactive solvent.

In at least one embodiment, the composition comprising a polyurethane pre-polymer (P) and a reactive diluent (F) may be a dispersion, a mixture or a combination of the polyurethane pre-polymer (P) and reactive diluent (F).

According to at least one embodiment, the polyurethane pre-polymer may be formed by reacting, in the presence of one or more di- or higher functionality (meth)acrylate monomers with optional presence of oligomers, acting as reactive diluent(s) (F), (reacting) of the following :
(A) one or more isocyanate-reactive ethylenically unsaturated components selected from polyester (meth)acrylates, epoxy (meth)acrylates, polyether (meth)acrylates, urethane (meth)acrylates or hydroxyl group-containing (meth)acrylates (meaning also hydroxyl-bearing (meth)acrylates), as defined above
with reactant components (B) and (C) as defined above.

According to at least one embodiment, component (A) may comprise additional non-(meth)acrylate free-radical polymerizable functionalities such as allylic or vinylic groups.

Component (A) comprises at least one (meth)acrylate functional group, in particular from 1 to 5, more particularly at least 2 or 3 (meth)acrylate groups. Component (A) comprises a monoalcohol (A1) of formula (1) bearing 3 (meth)acrylate groups and (A2) a diol of formula (2) bearing two (meth)acrylate groups, the said formulas being as shown below : 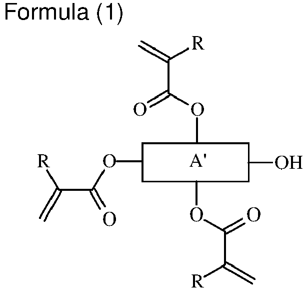 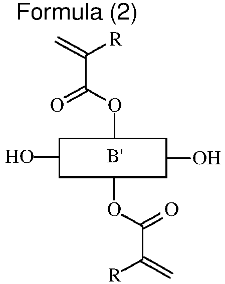 with R being either -H or -CH₃ and
wherein A' and B' represent the residues of corresponding polyols which are tetrols partially esterified by (meth)acrylic acid and which may be linear, cyclic or branched, substituted or unsubstituted hydrocarbon chains, wherein the optional substituents include cyclic groups and/or heteroatoms. Chains A' and B' may, for example, comprise an ester or ether group.

According to at least one embodiment, component (A) may be monomeric or oligomeric.

Suitable polyester (meth)acrylates suitable as (A) component, include, but are not limited to, the reaction products of acrylic or methacrylic acid or mixtures thereof with hydroxyl group terminated polyester polyols, where the reaction process is conducted such that a significant concentration of residual hydroxyl groups remain in the polyester (meth)acrylate. The polyester polyols can be di-, tri-, tetra-, penta- or higher in hydroxyl group functionality. The polyester polyols can be made by polycondensation reactions of di- or higher hydroxyl functional components with di- or higher functionality carboxylic acids or anhydrides. The hydroxyl functional and carboxylic acid components can each have linear, branched, cycloaliphatic or aromatic structures and can be used individually or as mixtures. Examples of suitable di- hydroxyl functional components include : 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, C₃₆-dimer diol, hydroquinone bis(2-hydroxyethyl) ether (HQEE), hydroxypivaloyl pivalate and ethoxylated and/or propoxylated derivatives of the above. Ethoxylated and/or propoxylated derivatives of bisphenol A or bisphenol F are also suitable. Suitable tri- and higher hydroxyl functional components include : glycerol, trimethylolpropane, trimethylolethane, pentaerythritol, di-glycerol, di-trimethyolpropane, di-pentaerytritol, sorbitol and ethoxylated and/or propoxylated derivatives of the above. Examples of suitable di- or higher functional carboxylic acids include : malonic acid, succinic acid, maleic acid, fumaric acid, itaconic acid, glutaric acid, adipic acid, pimelic acid, sebacic acid, dodecanedioic acid, phthalic acid, isophthalic acid, terephthalic acid, naphthalene dicarboxylic acid, dimer fatty acids, trimellitic acid, pyromellitic acid and the anhydride derivatives of the above. Suitable polyester polyols can also be made by ring opening polymerization of lactones initiated by a hydroxyl functional starter molecule such as those described above. Suitable lactones include alpha, alpha-dimethyl-beta-propiolactone, gamma-butyrolactone and epsilon-caprolactone. In particular, the said component (A) and (D) (see polyurethane polymer as defined above) are respectively derived and based on polyols, in particular on bio-based polyols, preferably on bio-based polyester polyols and/or polyether polyols or alkylene polyols.

More particularly, the hydroxyl group terminated polyester polyols used in accordance with embodiments of the present disclosure may comprise polyester biopolyols (same meaning as bio-based polyols from renewable resources). Some suitable examples of polyester biopolyols are given below for illustrating the invention, without any limitation to these examples.

In accordance with at least one embodiment, polyester polyols, in particular biopolyols may be made using renewable versions of unsaturated fatty acids, C₃₆ and C₅₄ dimer and trimer products, 1,4:3,6 dianhydrohexitols such as isosorbide and furan derivatives such as 2,5-furandicarboxylic acid. These and other exemplary polyols available from renewable resources are shown below.

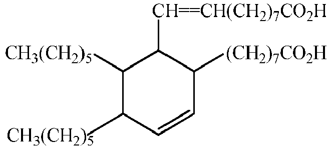

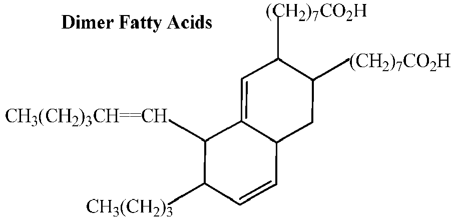

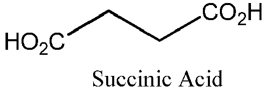

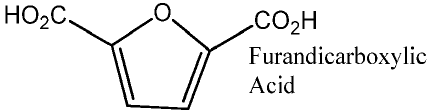

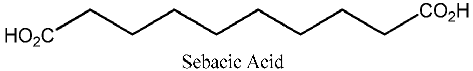

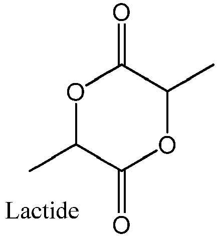

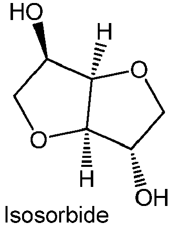

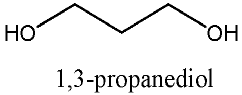

Examples of epoxy (meth)acrylates suitable as (A) component include the reaction products of acrylic or methacrylic acid or mixtures thereof with glycidyl ethers or esters. The glycidyl ethers or esters can have aliphatic, cycloaliphatic or aromatic structures and contain from two up to about six epoxy functional groups. Di-epoxy functional materials are preferred. Glycidyl ethers can be prepared from a hydroxyl functional precursor and an epoxy compound such as epichlorohydrin. Many of the hydroxyl functional components listed in the section above are suitable for preparation of aliphatic glycidyl ethers. Specific examples of precursors for aliphatic glycidyl ethers include : 1,4-butanediol, 2,2-dimethyl-1,3-propanediol, 1,6-hexanediol, 1,4- and 1,6-dimethylolcylcohexane, poly(ethylene glycol), polypropylene glycol), poly(tetramethylene glycol), trimethylolpropane, pentaerythritol, glycerol and sorbitol. Specific examples of precursors for aromatic glycidyl ethers include : bisphenol A, bisphenol F and resorcinol.

Examples of suitable polyether (meth) acrylates suitable as (A) component include the condensation reaction products of acrylic or methacrylic acid or mixtures thereof with polyetherols which are polyether polyols. Suitable polyetherols can be linear or branched substances containing ether bonds and terminal hydroxyl groups. Polyetherols can be prepared by ring opening polymerization of cyclic ethers such as tetrahydrofuran or alkylene oxides with a starter molecule. Suitable starter molecules include water, the hydroxyl functional materials as described above, polyester polyols and amines. Examples of suitable amines include : ethylene diamine, 4,4'-diaminodiphenylmethane, diethylene triamine and hydroxyl amines such as ethanol amine and diethanol amine. Examples of suitable alkylene oxides include : ethylene oxide, propylene oxide, butylenes oxides, epichlorohydrin and glycidol. The polyether (meth)acrylates can be used individually or in combination.

Examples of polyurethane (meth)acrylates (monomeric or oligomeric) suitable as (A) component include the polyaddition products of di- or poly-isocyanates as described below as component (B), with isocyanate-reactive ethylenically unsaturated components as described in the sections above as polyester-, epoxy- or polyether (meth)acrylates or immediately below-disclosed as monomeric hydroxyl containing (meth)acrylates and optionally the isocyanate-reactive components described below as component (D).

Examples of monomeric hydroxyl containing (meth)acrylates suitable as (A) component are acrylic, methacrylic or mixed esters of simple diols, triols, tetrols or polyols, where the esterification process is carried out such that residual hydroxyl groups remain in the final product. Examples include (meth)acrylate esters of : 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, glycerol, trimethylolpropane, trimethylolethane, pentaerythritol, di-glycerol, di-trimethyolpropane, di-pentaerytritol and sorbitol. The monomeric hydroxyl (meth)acrylates can be used individually or in mixtures.

Component (B) may comprise at least two isocyanate functional groups. In at least one embodiment, component (B) may comprise a diisocyanate having two isocyanate functional groups, such as, an aliphatic diisocyanate (e.g., isophorone diisocyanate). In other embodiments, component (B) may comprise a plurality of isocyanate groups, such as three or four or more isocyanate groups.

Non-limiting examples of compounds that may comprise component (B) include, di- or polyisocyanates such as aliphatic, aromatic and cycloaliphatic structures with at least two isocyanate functional groups per molecule. Examples of suitable isocyanate components (B) include : isophorone diisocyanate, hexamethylene diisocyanate, 2,3,3-trimethyl hexamethylene diisocyanate, 4,4'-dicylcohexylmethane diisocyanate, 1,5-naphthalene diisocyanate, 2,4- or 2,6-toluene diisocyanate and their isomeric mixtures, 4,4'-diphenylmethane diisocyanate. Polyisocyanates formed by creation of isocyanurate or biuret structures are also suitable as are mixtures of isocyanates. Polyisocyanates with allophanate modification may also be used as (B) component.

Without wishing to be bound by theory, it is believed that component (C) aids in the dispersion of the pre-polymer (self-dispersing after neutralization). Therefore, in at least one embodiment, the composition may not comprise a surfactant. In other embodiments, a surfactant may be added to better aid in the dispersion and its stability. Preferably, if a surfactant is used, it is a non-ionic surfactant.

In at least one embodiment, component (C) may comprise at least one acid functionality. For example, component (C) may comprise a polyol comprising an acid group selected from carboxylic (-CO₂H), sulfonic (-SO₃H), sulfonyl (-SO₂H), phosphoric (-OPO₃H₂), phosphonic (-PO₃H₂) and phosphinic (-PO₂H) acid groups. In at least one embodiment, component (C) comprises a diol having a carboxylic or sulfonic acid group.

Examples of component (C) include, but are not limited to, compounds containing at least one and preferably two isocyanate-reactive functional groups and at least one polar dispersive group which can be ionic, potentially ionic or polyether in character. Combinations of the different types can be used. Ionic or potentially ionic groups include carboxylic acid, sulfonic acid or phosphoric (ester) acid groups or their alkali metal or quaternary amine salts. If the free acid forms are used to prepare the pre-polymer (P), the acidic groups can be neutralized to the salt form before or during dispersion by addition of a base. Suitable bases include inorganic hydroxides or carbonates and amines and combinations. Specific examples of ionic/potentially ionic components with acidic nature include : 2-carboxy 1,3-propane diol, 2-sulfo 1,3-propane diol, 2-methyl-2-carboxy hexane diol, 3-methyl-3-carboxy hexane diol. 4-methyl-4-carboxy hexane diol. 2-ethyl-2-carboxy 1,3-propane diol. 2-ethyl-2-carboxy butane diol, hydroxyacetic acid, hydroxypropionic acid, malic acid, citric acid, dimethylolpropionic acid, dimethylolbutanoic acid, 2-sulfo-1,4-butanediol, 2,5-dimethyl-3-sulfo-2,5-hexanediol, 2-aminoethanesulfonic acid, N-(1,1-dimethyl-2-hydroxyethyl)-3-amino-2-hydroxypropanesulfonic acid, 2-aminoethylaminoethanesulfonic acid and salts of the above. Preferably, component (C) bears at least two isocyanate-reactive groups, such as OH groups.

Suitable polyether materials contain at least one preferably isocyanate-reactive group and a polyether chain such as those described above as possible precursors to polyether (meth)acrylates.

In at least one embodiment, component (D) is chosen from a monool or a polyol. According to at least one embodiment, component (D) may be added to increase the molecular weight of the polyurethane pre-polymer, to add additional functional groups to the polyurethane pre-polymer or to control the amount of terminal isocyanate groups on the polyurethane pre-polymer.

Examples of component (D) include, but are not limited to, one or more polyols with from one to about six isocyanate-reactive groups per molecule and number average molecular weight Mn from about 200 to 5000 Daltons. Suitable polyols as component (D) include polyesters, polyethers, polycarbonates, polycaprolactones, polybutadienes, hydrogenated polybutadienes, polyacrylics, polysiloxanes and fluorinated polyethers. Physical mixtures of the above or hybrid polyols with more than one structural type contained in the same molecule can be used.

According to at least one embodiment, component (D) may be monomeric or oligomeric.

When component (D) is used to form the polyurethane pre-polymer, the molar ratios of components (A), (B), (C) and (D) may be selected such that the number of isocyanate groups is in excess of the number of groups reactive with the isocyanate groups. That is, the isocyanate groups are in stoichiometric excess relative to the isocyanate-reactive groups issued from (A), (C) and (D). For example, when the isocyanate-reactive groups are hydroxy groups, the number of isocyanate groups is greater than the number of hydroxy groups. In at least one embodiment, the stoichiometric ratio of isocyanate groups to isocyanate-reactive groups is 1.01 to 3, such as from 1.1 to 2.5 or from 1.2 to 2.

Following the reaction of components (A), (C) and (D) with (B), all of the remaining isocyanate groups of the polyurethane pre-polymer (P) are reacted with a component (E) in a chain extension reaction, wherein component (E) is a chain extender with at least two isocyanate-reactive groups.

The reaction of the pre-polymer (P) with component (E) may occur before or after dispersion of the polyurethane pre-polymer (P) in water, by neutralization of the acidic groups. Preferably for viscosity purposes related with increase of viscosity with the molecular weight during extension reaction in solution, the dispersion occurs before the chain extension (lower viscosity in aqueous dispersion).

Non-limiting examples of chain extender component (E) include, but are not limited to, compounds selected from diamines, polyamines, primary or secondary amino-terminated polymers and mixtures thereof. Suitable diamines and polyamines can be linear, branched, aliphatic, cycloaliphatic or aromatic. Specific examples include ethylenediamine, 1,2-diaminopropane, 1,3-diaminopropane, 1,4-diaminobutane, 1,6-diaminohexane, 1,10-diaminodecane, 1,11-diamineundecane, 1,12-diaminododecane, dimer diamine, diethylenetriamine, triethylenetetramine, 4-azaheptamethylenediamine, N,N'-bis(3-aminopropyl)butane-1,4-diamine and mixtures thereof. Examples of primary or secondary amino-terminated polymers include polyamides, polyethylenimines, poly(vinylamines) and aminated polyalkylene oxides.

In accordance with at least one embodiment, the reaction to form the polyurethane pre-polymer (P), preferably comprise converting any potentially ionic groups into ionic groups by salt formation, by neutralization of the acidic groups beared by the (C) component. For example, in an embodiment where component (C) is dimethyolpropionic acid (DMPA), the hydroxyl groups may react with the isocyanate groups to incorporate the DMPA into the polyurethane chain while leaving the unreacted carboxylic acid group, which is a potentially ionic group. The carboxylic acid can be converted to a salt by addition of a base, such as, for example, an amine or inorganic base such as NaOH, KOH, NH₄OH, NaHCO₃ or the like.

In at least one embodiment, the reaction mixture for forming the polyurethane pre-polymer (P) may also comprise a catalyst and/or other additives, such as, for example, inhibitors, surfactants, fillers, stabilizers, photoinitiators, pigments, etc.

In accordance with at least one embodiment, the polyurethane pre-polymer is dispersed in water after neutralization.

In at least one embodiment, acid groups formed in the preparation of the polyurethane pre-polymer are neutralized prior to dispersion.

In at least one embodiment, the polyurethane pre-polymer (P) is formed in the absence of a non-reactive solvent. For example, the polyurethane pre-polymer may be formed in the absence of acetone.

According to at least one embodiment, the polyurethane pre-polymer (P) is formed in a reactive diluent (F). In at least one embodiment, the formed polyurethane pre-polymer (P) may be diluted in additional reactive diluent, which may be the same or different from the initial reactive diluent.

In accordance with at least one embodiment, the reactive diluent (F) comprises up to 90% by weight relative to the solids content of the dispersion. For example, the reactive diluent may comprise about 10% to about 90% by weight relative to the solids content of the dispersion. In other embodiments, the reactive diluent (F) may comprise about 20% to about 80% or from about 25% to about 75% or from about 25% to about 50% by weight relative to the solids content. As one of ordinary skill in the art would recognize, the amount of the reactive diluent (F) may be selected based on the desired viscosity of the dispersion, as well as the desired properties of the resulting polyurethane coating.

The polyurethane pre-polymer (P) formed by the reactions above after neutralization is dispersed in water to form an aqueous dispersion which is then used for the chain extension reaction with chain extender (E).

In at least one embodiment, the polyurethane pre-polymer (P) is diluted with a reactive diluent (F) prior to dispersing the polyurethane pre-polymer (P) in water. By dispersing the said polyurethane pre-polymer in water after diluting with the said reactive diluent, it may be possible to add a desired amount of said reactive diluent and then disperse the said polyurethane pre-polymer and obtain the desired viscosity by adding the water. In accordance with at least one embodiment, the polyurethane pre-polymer (P) is agitated as it is dispersed in water. In at least one embodiment, reactive diluent (F), either the same or different reactive diluent if already present, can be added to the aqueous polyurethane polymer dispersion.

The reactive diluent (F) may be selected to provide desired properties of the polyurethane. For example, the reactive diluent may be selected to adjust the properties of the polyurethane, such as the hardness, weatherability, texture, abrasion resistance, flexibility and the like.

In accordance with at least one embodiment, the reactive diluents are materials with two or more ethylenically unsaturated groups, such as, for example, (meth)acrylate groups. The reactive diluents (F) can be monomeric or oligomeric and can be used individually or in combination. When used as reactive diluents rather than as components of the pre-polymer, the hydroxyl group content is not critical. Suitable monomeric examples include the (meth)acrylate esters of 1,2-ethanediol, 1,2-propanediol, 1,3-propanediol, 1,4-butanediol, 1,3-butanediaol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, tetraethylene glycol, dipropylene glycol, tripropylene glycol, 1,4- and 1,6-dimethylolcylcohexane, glycerol, trimethylolpropane, trimethylolethane, pentaerythritol, di-glycerol, di-trimethyolpropane, di-pentaerytritol, sorbitol and alkoxylated derivatives of the above. Many such materials are available commercially from Sartomer as SR-coded products.

Oligomeric reactive diluents (F) include the polyester-, polyether- or urethane (meth)acrylates as described above as components (A) of the pre-polymer (P), except that, in the use as a reactive diluent (F), there is none free hydroxyl group and in general none isocyanate-reactive group. Many such products are available commercially from Sartomer as "CN" products and fulfilling the above definition.

The polyurethane pre-polymer (P) is subjected to the chain extension reaction with extender (E) in order to form the final polyurethane polymer. The chain extender may be selected to alter the hardness, weatherability, flexibility or adhesiveness. The chain extenders may be selected from polyols and polyamines, such as, for example, diols and diamines. In at least one embodiment, the chain extender is selected from diamines.

The chain extender (E) comprises two or more functional groups reactive with the isocyanate terminal groups of the said polyurethane pre-polymer (P). In at least one embodiment, the chain extender comprises two isocyanate-reactive functional groups and functions to extend the polyurethane chain. In at least one embodiment, a mixture of chain extenders comprising two functional groups and three or more functional groups may be used.

As used herein, the phrase "substantially all of the terminal isocyanate groups" and variations thereof means that at least 95% of the terminal isocyanate groups are involved in the chain extension reaction, such as for example, at least 98% or at least 99% of the terminal isocyanate groups, preferably 100%.

In accordance with at least one embodiment, the polyurethane is dispersed in water prior to the chain extension reaction.

The polyurethane dispersion is preferably free of non-reactive solvent. According to at least one embodiment, the polyurethane pre-polymer (P) has a number average molecular weight Mn ranging from about 400 to about 15 000 Daltons, such as, for example, from about 700 to about 10 000 Daltons. Following chain extension, the polyurethane polymer may have a number average molecular weight Mn ranging from about 1500 to about 60 000 Daltons, such as, for example, from about 2000 to about 50 000 Daltons. The molecular weights Mn are determined by gel permeation chromatography (GPC) in THF using polystyrene calibration standards. The molecular weight and polydispersity are thus determined by conventional gel permeation chromatography (GPC). A small sample is dissolved in tetrahydrofuran (THF) and injected into a liquid chromatograph (Agilent 1100 Series) equipped with HP PLGel^{®} GPC columns (5 um, 100A, 250 x 4.6 mm ; 3 um MiniMix-E, 250 x 4.6 mm and 5 um MiniMix-D, 250 x 4.6 mm). The components of the sample are separated by the GPC columns based on their molecular sizes in solution. The components are detected by an Agilent 1047A^{®} refractive index detector and recorded by Agilent HPLC Chemstation^{®} and Polymer Laboratories GPC software. Polystyrene standards of known molecular weight and narrow dispersity are used to generate a calibration curve.

In at least one more particular embodiment, the present disclosure relates to the use in a nail polish composition of a polyurethane polymer aqueous dispersion which is a solvent-free dispersion and comprises a polyurethane polymer bearing polymerizable ethylenically unsaturated pending (side-) and terminal (end-) groups in particular (meth)acrylate side- and end-groups. More particularly, in this case :
- said component (A) is a polyol component comprising :
   (A1) at least one monool bearing at least one ethylenically unsaturated group, preferably a (meth)acrylate monool with a (meth)acrylate functionality of at least 1, more preferably of at least 2,
   (A2) at least one diol bearing at least one ethylenically unsaturated group, preferably a (meth)acrylate diol with a (meth)acrylate functionality of at least 1
- said component (B) is a polyisocyanate component comprising at least one polyisocyanate bearing at least 2 isocyanate (NCO) groups, preferably from 2 to 3, and more preferably 2 isocyanate groups
- said component (C) is at least one diol bearing an acid group, preferably selected from carboxy or sulfonic or phosphoric or phosponic or phosphinic groups, more preferably carboxy or sulfonic groups
- said component (D) is a saturated diol different from (A2), which is a monomeric or an oligomeric diol, preferably an oligomeric diol, in particular with Mn < 1000, more preferably a polyester and/or polyether diol. Preferably, the reaction of (A), (C) and (D) with (B) is under conditions of excess of NCO groups with respect to the total of OH groups and preferably with NCO/OH (total) ratio being from 1.01 to 3, preferably from 1.1 to 2.5 and more preferably from 1.2 to 2 and the resulting NCO-ended pre-polymer (P) from (A), (C) and (D) reaction with (B), is then chain extended by additional chain extension reaction of NCO-terminal groups of (P) with a chain extender (E) bearing 2 isocyanate-reactive groups, (E) preferably being selected from diols and/or diamines, more preferably diamines, with in particular in last case an urea group content of at least 1 mmol per kg of dry polymer, the said acidic groups of said polyurethane pre-polymer (P) being at least partly neutralized, preferably completely neutralized by a weak base, more preferably selected from amines or phosphines, even more preferably from amines and more particularly from tertiary amines before dispersion in water and chain extending with (E).

A specific process of preparing the said particular aqueous polyurethane polymer dispersion with the polymer having both pending and terminal ethylenically unsaturated groups is by a solvent-free process comprising the following successive steps :
i) preparing an NCO-ended pre-polymer (P) by reacting the polyol components (A), (C) and (D) with polyisocyanate (B) as discussed above ;
ii) neutralizing at least partly, preferably completely, the said acid groups ;
iii) dispersing the neutralized pre-polymer of step ii) in water under agitation to obtain an aqueous dispersion of said pre-polymer (P) ; and
iv) adding a chain extender (E) in the dispersion, which extender (E) bears two groups isocyanate-reactive groups, the extender preferably selected from diols and/or diamines, more preferably from diamines, so that the said pre-polymer (P) is chain-extended ; and
v) optionally, and if needed, adjusting the solids content by dilution or adjusting the pH for obtaining the aqueous polyurethane polymer as defined above according to the particular embodiment of the present invention.

According to at least one embodiment of the present disclosure, the nail polish composition comprising the said aqueous polyurethane polymer dispersion a) as defined above according to the present invention, is radiation-curable. In at least one embodiment, it may be cured by exposure to actinic radiation. According to at least one embodiment, it is cured by exposure to ultraviolet light, LED or visible light.

More particularly, said dispersions are used for coating nails. The object may be coated with the said polyurethane dispersion, which is subsequently cured by actinic radiation.

The polyurethane dispersions of the present disclosure may also be used to coat objects, such as for example, wood, metal, plastic, ceramic, composite objects, glass, fibers, textiles, leather, stone, concrete and other materials. The polyurethane dispersions of the present disclosure may be used to form coatings that provide protection against mechanical, chemical and/or environmental effects. The said polyurethane dispersions may also be used as adhesives, surface modifiers, surface coatings and inks. The polyurethane dispersions of the present disclosure may also be used for adhesives, sealants, inks and other applications, such as providing surface texture or haptic effects.

More preferably, the polyurethane dispersions of the present disclosure are used for cosmetic applications, including in particular nail coatings.

The polyurethane coatings formed from polyurethane dispersions of the present disclosure may be used to provide scratch, abrasion and wear resistance ; UV protection ; corrosion resistance ; surface appearance, such as a glossy or flat appearance ; chemical and stain resistance ; hydrolytic resistance ; flame retardancy ; anti-microbial activity ; electrical conduction or insulation ; barrier or permeability to gasses ; adhesion ; haptic effects such as soft touch ; easy cleaning and anti-fingerprint. The properties of the resultant polyurethane coatings may be controlled by varying the amounts of the components present within the polyurethane dispersions described above.

The present invention particularly relates to the use of the aqueous polyurethane dispersions disclosed herein as or in nail polish formulations. The aqueous polyurethane dispersions described above may be formulated for application to the nails. The actinic radiation curable polyurethanes may be cured upon exposure to sunlight or UV/LED lamps to create a hard and glossy/shiny nail varnish or coating.

According to a particular option of the present invention, the said nail polish composition is a nail polish formulation and in addition to said aqueous polyurethane polymer dispersion said formulation further comprises :
b) a photoinitiator
c) optionally, a leveling agent and
d) optionally, a thickener,
and wherein said formulation is free of non-reactive solvents.

The said formulation may further comprise e) at least one coloring agent.

Said formulation is UV- curable, in particular by LED or visible light.

It may have a solids level of 20% to 30% and a viscosity of 500 to 3000 mPa.s at room temperature. Room temperature means at about 25°C.

The present disclosure does also relate to the use of said nail polish formulation for coating nails (or varnishing nails). It also relates to the use of any of the aqueous, actinic radiation-curable polyurethane polymer dispersions as defined above according to the invention, in a nail polish formulation. Also, they are particularly used for nail coating or varnishing.

The dislcosure does also relate to a cured coating or varnish, which is a nail coating or varnish (coating or varnish applied and cured on nails) and which coating or varnish results from the UV cure of at least one nail polish composition or cure of at least one nail polish formulation as defined above according to the present invention.

In at least one particular embodiment, the nail polish composition comprises, consists essentially of or consists of, an aqueous polyurethane polymer dispersion a) of the invention, b) a photoinitiator and c) optionally a leveling agent and/or d) optionally a thickener. A leveling agent c) may be included to aid in the formation of a smooth and homogeneous surface of the coating. The leveling agent may be selected from any known leveling agent for use in nail polish formulations, such as, for example, BYK^{®}-346 (available from BYK-CHEMIE GMBH).

Thickeners may be added to the nail polish formulation to adjust the viscosity of the formulation and/or to control the flow properties of the formulation. Non-limiting examples of thickeners include Acrysol^{®} RM-825, Aculyn^{®} 33 and Aculyn^{®} 44 (available from Dow Chemical Co.).

In accordance with at least one embodiment, the nail polish formulation has a viscosity ranging from about 250 mPa.s (cP) to about 4000 mPa.s (cP), such as from about 500 mPa.s (cP) to about 3000 mPa.s (cP), preferably from about 1000 mPa.s (cP) to 2000 mPa.s (cP), at room temperature (25°C). The said viscosity is a Brookfield viscosity measured at 25°C at 100 rpm.

According to at least one embodiment, the nail polish formulation has a solids level ranging from about 10% to about 40%, preferably from about 20% to about 30%, for example about 25%.

In at least one embodiment, the nail polish formulation may also and further comprise e) one or more coloring agents, such as a pigment, dyes or other colorants and may also include solid particulates or granulates to be applied as part of the nail coating. The nail polish formulation may further comprise fillers, surfactants, stabilizers, fragrances and/or preservatives.

According to at least one embodiment, the nail polish formulation is free or substantially free of any non-reactive solvents. In the description of the embodiments and examples herein, the transitional phrase "comprising" has been used. However, the invention is also understood to include embodiments consisting of and consisting essentially of the components described for each embodiment.

### EXAMPLES

### Example 1

A reactive diluent (SR454, available from Sartomer), dimethylol propionic acid (DMPA), an inhibitor (IRGANOX^{®} 1035), a catalyst (REAXIS^{®} C716) and isophorone diisocyanate (IPDI) were charged to a reactor and agitated. The reaction mixture was heated to 70°C until the desired isocyanate group (% NCO) content was reached.

A polycarbonate polyol (OXYMER^{®} M112), an epoxy (meth)acrylate (CN104Z, available from Sartomer) and a hydroxyl containing (meth)acrylate were then added to the reactor and the mixture heated to 70°C until the desired % NCO was reached.

Triethylamine was then added to neutralize remaining acidic groups.

Water and ethylene diamine (EDA) were then added to the polyurethane pre-polymer to disperse and chain-extend the pre-polymer, forming the aqueous polyurethane dispersion.

### Example 2

### Nail Polish Composition

97.73 wt parts of the polyurethane dispersion of example 1, which has been diluted with water to 35% solids, is mixed with 0.17 wt part of a leveling agent (BYK^{®}-346, available from BYK-CHEMIE GMBH), 1.74 wt part of a photoinitiator (PL-4265 from PL Industries) and about 0.35 wt part of a thickener Acrysol^{®} RM825 to form a nail polish formulation (100 wt parts).

The formulation is coated on aluminum and cured under UV energy of 1 J/cm². The measured 60° gloss is 170.

The formulation was also applied to acrylic artificial nails using a brush applicator. Once the water evaporated, a homogeneous film was formed without brush marks. After curing under UV energy of 1 J/cm², the formulation produced a smooth, homogeneous and shiny film. The adhesion of the nail polish formulation on the acrylic artificial nails was excellent and could not be peeled or cracked off using finger nails.

## Claims

1. Use in nail polish composition, in particular nail polish formulation, of at least one aqueous, actinic radiation curable polyurethane dispersion, wherein said dispersion is free of non-reactive solvent and comprises a polyurethane polymer in at least one reactive diluent (F) and the said polyurethane polymer, in particular bearing ethylenic unsaturation end-groups and optionally side groups, is formed by chain extending at least one isocyanate-terminated ethylenically unsaturated polyurethane pre-polymer (P), in particular with at least one chain extender (E) bearing at least 2 isocyanate-reactive groups and wherein said pre-polymer is formed by reacting :
(A) one or more isocyanate-reactive components containing at least one ethylenic unsaturation chosen from active hydrogen-containing (meth)acrylates ;
(B) one or more polyisocyanates, preferably diisocyanates ;
(C) one or more isocyanate reactive components containing ionic groups, potentially ionic groups or hydrophilic ether groups, preferably ionic groups derived from acidic groups, in particular with said component (C) bearing two isocyanate-reactive groups, more particularly bearing two OH groups ; and
(D) optionally, one or more isocyanate-reactive components other than component (A) or component (C), preferably bearing two isocyanate-reactive groups, in particular OH groups,
such that the mole ratios of components (A), (B), (C) and (D) result in a polyurethane pre-polymer comprising terminal isocyanate group and
said component (A) comprising a monoalcohol (A1) of formula (1) bearing 3 (meth)acrylate groups and (A2) a diol of formula (2) bearing two (meth)acrylate groups, the said formulas being as shown below : 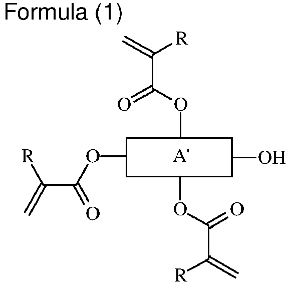 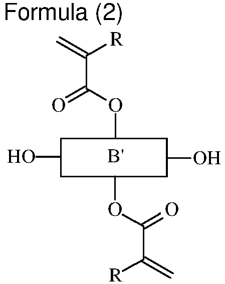 with R being either -H or -CH₃ and
wherein A' and B' represent the residues of corresponding polyols which are tetrols partially esterified by (meth)acrylic acid and which may be linear, cyclic or branched, substituted or unsubstituted hydrocarbon chains, wherein the optional substituents include cyclic groups and/or heteroatoms.

2. Use of claim 1, wherein said dispersion is an actinic radiation-curable dispersion, more preferably the actinic radiation being : UV, LED, visible light radiation.

3. The use of claim 1 or 2, wherein the said one or more isocyanate-reactive components (A) containing ethylenic unsaturation is chosen from polyester (meth)acrylates, epoxy (meth)acrylates, polyether (meth)acrylates, urethane (meth)acrylates, hydroxy-bearing (meth)acrylates and combinations thereof.

4. The use of any one of claims 1 to 3, wherein the said component (C) is a diol bearing an ionic group resulting from an acidic group chosen from carboxylic,
preferably carboxylic or sulfonic groups, with said acidic group being at least partly neutralized with a basic agent.

5. The use of any one of claims 1 to 4, wherein the polyurethane pre-polymer is further reacted with a component (E), comprising at least two isocyanate reactive groups, preferably selected from diamines.

6. The use of any one of claims 1 to 5, wherein the said reactive diluent (F) represents from 10 to 90%, preferably from 10 to 50% by weight relative to the solids content of the dispersion.

7. The use of any one of claims 1 to 6, wherein said components (A) and (D) as defined in claim 1, are respectively derived and based on polyols, in particular on bio-based polyols, preferably on bio-based polyester polyols and/or polyether polyols or alkylene polyols.

8. The use of any one of claims 1 to 7, wherein said polyurethane polymer aqueous dispersion a) is a solvent-free dispersion and comprises a polyurethane polymer bearing polymerizable ethylenically unsaturated pending (side-) groups and terminal (end-) groups, in particular (meth)acrylate side-groups and end-groups.

9. The use of any one of claims 1 to 8, wherein said nail composition is a nail polish formulation and in addition to said aqueous polyurethane polymer dispersion said formulation further comprises :
b) a photoinitiator
c) optionally, a leveling agent and
d) optionally, a thickener,
and wherein said formulation is free of non-reactive solvents.

10. The use of claim 9, wherein it further comprises e) at least one coloring agent.

11. The use of claim 9 or 10, wherein the said formulation is UV- curable.

12. The use of any one of claims 9 to 11, wherein the said formulation has a solids level of 20% to 30% and a Brookfield viscosity of 500 to 3000 mPa.s (cP) measured at 25°C at 100 rpm.

13. The use according to any one of claims 1 to 12, wherein said nail polish formulation is UV-curable.

## Patentansprüche

1. Verwendung von mindestens einer wässrigen, mit aktinischer Strahlung härtbaren Polyurethandispersion in einer Nagellackzusammensetzung, insbesondere einer Nagellackformulierung, wobei die Dispersion frei von nichtreaktivem Lösungsmittel ist und ein Polyurethanpolymer in mindestens einem reaktiven Verdünnungsmittel (F) umfasst und das Polyurethanpolymer, das insbesondere ethylenisch ungesättigte Endgruppen und gegebenenfalls Seitengruppen trägt, durch Kettenverlängerung mindestens eines isocyanatterminierten ethylenisch ungesättigten Polyurethanprepolymers (P) gebildet wird, insbesondere mit mindestens einem Kettenverlängerungsmittel (E), das mindestens 2 gegenüber Isocyanat reaktive Gruppen trägt, und wobei das Prepolymer gebildet wird durch Umsetzen von:
(A) einer oder mehreren gegenüber Isocyanat reaktiven Komponenten mit mindestens einer ethylenischen Ungesättigtheit, die aus aktiven Wasserstoff enthaltenden (Meth)acrylaten ausgewählt sind;
(B) einem oder mehreren Polyisocyanaten, vorzugsweise Diisocyanaten;
(C) einer oder mehreren gegenüber Isocyanat reaktiven Komponenten mit ionischen Gruppen, potentiell ionischen Gruppen oder hydrophilen Ethergruppen, vorzugsweise ionischen Gruppen, die sich von sauren Gruppen ableiten, wobei die Komponente (C) insbesondere zwei gegenüber Isocyanat reaktive Gruppen und weiter bevorzugt zwei OH-Gruppen trägt; und
(D) gegebenenfalls einer oder mehreren gegenüber Isocyanat reaktiven Komponenten, die von Komponente (A) oder Komponente (C) verschieden sind und vorzugsweise zwei gegenüber Isocyanaten reaktive Gruppen, insbesondere OH-Gruppen, tragen,
derart, dass die Molverhältnisse der Komponenten (A), (B), (C) und (D) zu einem Polyurethanprepolymer mit endständigen Isocyanatgruppen führen, und
wobei die Komponente (A) einen Monoalkohol (A1) der Formel (1), der 3 (Meth)acrylatgruppen trägt, und (A2) ein Diol der Formel (2), das 2 (Meth)acrylatgruppen trägt, umfasst, wobei die Formeln wie nachstehend gezeigt sind: 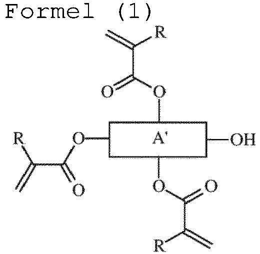 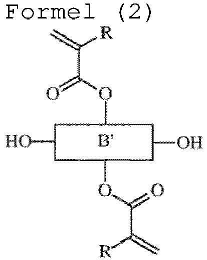 wobei R entweder für -H oder -CH₃ steht und
wobei A' und B' für die Reste entsprechender Polyole, bei denen es sich um teilweise mit (Meth)acrylsäure veresterte Tetrole handelt, stehen, bei denen es sich um lineare, cyclische oder verzweigte, substituierte oder unsubstituierte Kohlenwasserstoffketten handeln kann, wobei die fakultativen Substituenten cyclische Gruppen und/oder Heteroatome einschließen.

2. Verwendung nach Anspruch 1, wobei es sich bei der Dispersion um eine mit aktinischer Strahlung härtbare Dispersion handelt, wobei es sich weiter bevorzugt bei der aktinischen Strahlung um UV-, LED-, sichtbare Lichtstrahlung handelt.

3. Verwendung nach Anspruch 1 oder 2, wobei die eine oder die mehreren gegenüber Isocyanat reaktiven Komponenten (A) mit ethylenischer Ungesättigtheit aus Polyester(meth)acrylaten, Epoxy(meth)acrylaten, Poly-ether-(meth)acrylaten, Urethan(meth)acrylaten, hydroxylgruppenhaltigen (Meth)acrylaten und Kombinationen davon ausgewählt sind.

4. Verwendung nach einem der Ansprüche 1 bis 3, wobei es sich bei der Komponente (C) um ein Diol handelt, das eine aus einer sauren Gruppe, die aus Carbonsäuregruppen, vorzugsweise Carbonsäure- oder Sulfongruppen, ausgewählt ist, resultierende ionische Gruppe trägt, wobei die Säuregruppe mindestens teilweise mit einem basischen Mittel neutralisiert ist.

5. Verwendung nach einem der Ansprüche 1 bis 4, wobei das Polyurethanprepolymer ferner mit einer Komponente (E) mit mindestens zwei gegenüber Isocyanat reaktiven Gruppen, die vorzugsweise aus Diaminen ausgewählt ist, umgesetzt wird.

6. Verwendung nach einem der Ansprüche 1 bis 5, wobei das reaktive Verdünnungsmittel (F) 10 bis 90 Gew.-%, vorzugsweise 10 bis 50 Gew.-%, bezogen auf den Feststoffgehalt der Dispersion, ausmacht.

7. Verwendung nach einem der Ansprüche 1 bis 6, wobei die wie in Anspruch 1 definierten Komponenten (A) und (D) sich jeweils von Polyolen, insbesondere biobasierten Polyolen, vorzugsweise biobasierten Polyesterpolyolen und/oder Polyetherpolyolen oder Alkylenpolyolen, ableiten und darauf basieren.

8. Verwendung nach einem der Ansprüche 1 bis 7, wobei die wässrige Polyurethanpolymerdispersion a) eine lösungsmittelfreie Dispersion ist und ein Polyurethanpolymer, das polymerisierbare ethylenisch ungesättigte anhängende Gruppen (Seitengruppen) und endständige Gruppen (Endgruppen), insbesondere (Meth)acrylat-Seitengruppen und -Endgruppen, trägt, umfasst.

9. Verwendung nach einem der Ansprüche 1 bis 8, wobei es sich bei der Nagelzusammensetzung um eine Nagellackformulierung handelt und die Formulierung zusätzlich zu der wässrigen Polyurethanpolymerdispersion ferner Folgendes umfasst:
b) einen Photoinitiator,
c) gegebenenfalls ein Verlaufmittel und
b) gegebenenfalls einen Verdicker,
und wobei die Formulierung frei von nichtreaktiven Lösungsmitteln ist.

10. Verwendung nach Anspruch 9, wobei sie ferner e) mindestens ein Farbmittel umfasst.

11. Verwendung nach Anspruch 9 oder 10, wobei die Formulierung UV-härtbar ist.

12. Verwendung nach einem der Ansprüche 9 bis 11, wobei die Formulierung einen Feststoffgehalt von 20 % bis 30 % und eine bei 25 °C bei 100 U/min gemessene Brookfield-Viskosität von 500 bis 3000 mPa.s (cP) aufweist.

13. Verwendung nach einem der Ansprüche 1 bis 12, wobei die Nagellackformulierung UV-härtbar ist.

## Revendications

1. Utilisation dans une composition de vernis à ongles, en particulier une formulation de vernis à ongles, d'au moins une dispersion aqueuse, durcissable par un rayonnement actinique, de polyuréthane, ladite dispersion étant exempte d'un solvant non réactif et comprenant un polymère de type polyuréthane dans au moins un diluant réactif (F) et ledit polymère de type polyuréthane, en particulier portant des groupes terminaux et éventuellement des groupes latéraux, à insaturation éthylénique, étant formé par extension de chaînes d'au moins un prépolymère (P) de type polyuréthane éthyléniquement insaturé terminé par isocyanate, en particulier avec au moins un agent d'extension de chaînes (E) portant au moins 2 groupes réactif envers un isocyanate et ledit prépolymère étant formé par mise en réaction :
(A) d'un ou plusieurs composants réactifs envers un isocyanate contenant au moins une insaturation éthylénique choisis parmi des (méth)acrylates contenant un hydrogène actif ;
(B) d'un ou plusieurs polyisocyanates, préférablement des diisocyanates ;
(C) d'un ou plusieurs composants réactifs envers un isocyanate contenant des groupes ioniques, des groupes potentiellement ioniques ou des groupes éther hydrophiles, préférablement des groupes ioniques issus de groupes acides, en particulier ledit composant (C) portant deux groupes réactifs envers un isocyanate, plus particulièrement portant deux groupes OH ; et
(D) éventuellement, d'un ou plusieurs composants réactifs envers un isocyanate autres que le composant (A) ou le composant (C), préférablement portant deux groupes réactifs envers un isocyanate, en particulier des groupes OH,
de sorte que les rapports molaires de composants (A), (B), (C) et (D) résultent en un prépolymère de type polyuréthane comprenant un groupe isocyanate terminal et
ledit composant (A) comprenant un monoalcool (A1) de formule (1) portant 3 groupes (méth)acrylate et (A2) un diol de formule (2) portant deux groupes (méth)acrylate, lesdites formules étant telles que présentées ci-dessous : 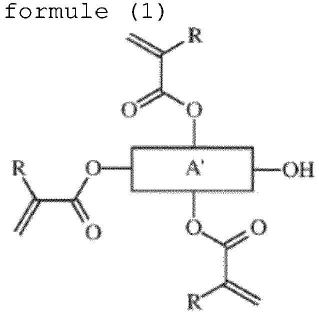 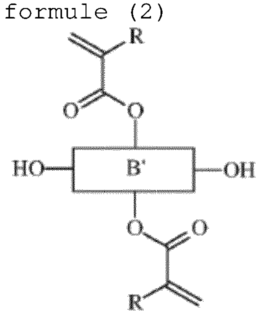 R étant soit -H, soit -CH₃ et
A' et B' représentant les résidus de polyols correspondants qui sont des tétrols partiellement estérifiés par un acide (méth)acrylique et qui peuvent être des chaînes hydrocarbonées linéaires, cycliques ou ramifiées, substituées ou non substituées, les substituants éventuels comprenant des groupes cycliques et/ou des hétéroatomes.

2. Utilisation selon la revendication 1, ladite dispersion étant une dispersion durcissable par un rayonnement actinique, plus préférablement le rayonnement actinique étant : un rayonnement UV, un rayonnement LED, un rayonnement de lumière visible.

3. Utilisation selon la revendication 1 ou 2, ledit ou lesdits composants réactifs envers un isocyanate (A) contenant une insaturation éthylénique étant choisis parmi des (méth)acrylate de polyester, des (méth)acrylates d'époxy, des (méth)acrylates de polyéther, des (méth)acrylates d'uréthane, des (méth)acrylates portant un hydroxy et des combinaisons correspondantes.

4. Utilisation selon l'une quelconque des revendications 1 à 3, ledit composant (C) étant un diol portant un groupe ionique résultant d'un groupe acide choisi parmi des groupes carboxylique, préférablement carboxyliques ou sulfoniques, ledit groupe acide étant au moins partiellement neutralisé par un agent basique.

5. Utilisation selon l'une quelconque des revendications 1 à 4, le prépolymère de type polyuréthane étant en outre mis à réagir avec un composant (E), comprenant au moins deux groupes réactifs envers un isocyanate, préférablement choisi parmi des diamines.

6. Utilisation selon l'une quelconque des revendications 1 à 5, ledit diluant réactif (F) représentant de 10 à 90 %, préférablement de 10 à 50 % en poids par rapport à la teneur en solides de la dispersion.

7. Utilisation selon l'une quelconque des revendications 1 à 6, lesdits composants (A) et (D) tels que définis dans la revendication 1, étant respectivement issus et à base de polyols, en particulier de polyols d'origine biologique, préférablement à base de polyester polyols et/ou de polyéther polyols ou d'alkylène polyols d'origine biologique.

8. Utilisation selon l'une quelconque des revendications 1 à 7, ladite dispersion aqueuse de polymère de type polyuréthane a) étant une dispersion exempte de solvant comprenant un polymère de type polyuréthane portant des groupes (latéraux) pendants et des groupes (terminaux) terminaux, éthyléniquement insaturés polymérisables, en particulier des groupes latéraux et des groupes terminaux de type (méth)acrylate.

9. Utilisation selon l'une quelconque des revendications 1 à 8, ladite composition pour ongles étant une formulation de vernis à ongles et, en plus de ladite dispersion aqueuse de polymère de type polyuréthane, ladite formulation comprenant en outre :
b) un photoinitiateur
c) éventuellement, un agent nivelant et
d) éventuellement, un épaississant,
et ladite formulation étant exempte de solvants non réactifs.

10. Utilisation selon la revendication 9, qui comprend en outre e) au moins un agent colorant.

11. Utilisation selon la revendication 9 ou 10, ladite formulation étant durcissable par des UV.

12. Utilisation selon l'une quelconque des revendications 9 à 11, ladite formulation possédant un taux de solides de 20 % à 30 % et une viscosité Brookfield de 500 à 3 000 mPa.s (cP) mesurée à 25 °C à 100 tpm.

13. Utilisation selon l'une quelconque des revendications 1 à 12, ladite formulation de vernis à ongles étant durcissable par des UV.
